**Europäisches Patentamt**

**European Patent Office**

**Office européen des brevets**

(11) Veröffentlichungsnummer: **0 351 659 B1**

(12) **EUROPÄISCHE PATENTSCHRIFT**

(45) Veröffentlichungstag der Patentschrift: **10.02.93**

(21) Anmeldenummer: **89112378.8**

(22) Anmeldetag: **06.07.89**

(51) Int. Cl.⁵: **G01N 21/21**, G01J 4/04

(54) **Verfahren zur Messung der Konzentration optisch aktiver Substanzen und Anordnung zur Durchführung des Verfahrens.**

(30) Priorität: **19.07.88 DE 3824589**

(43) Veröffentlichungstag der Anmeldung:
**24.01.90 Patentblatt 90/04**

(45) Bekanntmachung des Hinweises auf die Patenterteilung:
**10.02.93 Patentblatt 93/06**

(84) Benannte Vertragsstaaten:
**DE FR GB IT SE**

(56) Entgegenhaltungen:
**DE-A- 2 734 391**
**DE-B- 1 121 357**
**GB-A- 882 244**
**GB-A- 2 197 467**
**US-A- 3 397 607**

**OPTICS AND SPECTROSCOPY, Band 32, Nr. 6, Juni 1972, Seiten 667-669 ; A.V. MALAK-HOVSKII : "Automatic Spectropolarimeter".**

(73) Patentinhaber: **SIEMENS AKTIENGESELL-SCHAFT**
**Wittelsbacherplatz 2**
**W-8000 München 2(DE)**

(72) Erfinder: **Paul, Bernt-Joachim, Dr.**
**Emil-Kränzlein-Strasse 8b**
**W-8520 Erlangen(DE)**

**Beschreibung**

Die Erfindung bezieht sich auf ein Verfahren und eine Anordnung zur Messung der Konzentration optisch aktiver Substanzen, insbesondere der Glucosekonzentration in Körperflüssigkeiten, durch Polarisationsmessung mit polarisiertem Licht einer Lichtquelle. Das Licht wird über einen Modulator, der mit einer vorbestimmten Modulationsfrequenz betrieben wird, sowie eine Meßküvette, welche die Substanz enthält, und einen Analysator einem Detektor zugeführt.

Ein bekanntes Polarimeter arbeitet nach dem Prinzip des automatischen, optischen Nullabgleichs. Die Anordnung enthält Zwei Linienstrahler, eine Quecksilber- und eine Natriumdampflampe sowie fünf auf einem Filterrad angeordnete optische Filter. Durch einen mit der Filterradachse gekoppelten Schwenkspiegel wird bei der Wahl der Meßwellenlänge auch zugleich die Strahlung der entsprechenden Lichtquelle in den Polarimeterstrahlengang eingespiegelt. Ferner ist ein Gitter-Monochromator und eine Quarz-Jod- sowie eine Deuterium-Lampe vorgesehen, die beide eine kontinuierliche Strahlung liefern. Am Monochromator läßt sich die gewünschte Wellenlänge auswählen. In dieser bekannten Ausführungsform gelangt das monochromatische Licht durch den Polarisator, die Küvette mit der Probe und den Analysator auf einen Photomultiplier. Polarisator und Analysator werden von je einem drehbar angeordneten Glan-Prisma aus Kalkspat gebildet. Der Polarisator und damit auch die Polarisationsebene des durchtretenden Lichtes schwingen mit einer Auslenkung von etwa ± 0,7 Winkelgrad mit 50 Hz um die optische Längsachse des Systems. Dadurch entsteht im unabgeglichenen Zustand des Systems im Photomultiplier ein 50 Hz-Signal, das verstärkt und vorzeichenrichtig einem Servomotor zugeführt wird, der mechanisch mit dem Analysator verbunden ist und den Analysator solange dreht, bis das 50 Hz-Signal Null wird. Eine in den Strahlengang gebrachte optisch aktive Probe dreht die Polarisationsebene, und der Analysator wird durch das Servosystem in die neue Abgleichstellung nachgeführt. Die Winkeldifferenz zwischen dieser neuen und der ursprünglichen Abgleichstellung entspricht dem optischen Drehvermögen der Probe. Diese Einrichtung mit mechanischer Bewegung des Polarisators und damit einem mechanisch wirkenden Modulator sowie motorischer Drehung des Analysators ist für eine Miniaturisierung und somit für eine Implantierung ungeeignet (Perkin Elmer 241 MC).

Es ist ferner ein Verfahren zur Bestimmung der Blutglucose durch Polarimetrie bekannt, bei dem polarisiertes Licht über einen Faraday-Modulator, der mit einer vorbestimmten Modulationsfrequenz betrieben wird, sowie über die Probe und einen Analysator, einem Detektor zugeführt wird. Bei diesem Verfahren erfolgt eine Strahlaufteilung in ein Meß- und ein Referenzsignal, für das ein weiterer Detektor vorgesehen ist. Anschließend wird aus den Ausgangssignalen der beiden Detektoren der Quotient gebildet. Ein Verfahren mit Strahlteilung erfordert jedoch einen entsprechenden Aufwand für die Optik; insbesondere müssen an die beiden Detektoren Gleichlaufanforderungen gestellt werden (DE-OS 29 44 113).

Eine bekannte Vorrichtung zum Nachweis von Schwebeteilchen arbeitet mit polarisiertem Licht, das an den Schwebeteilchen gestreut und einem Detektor zugeführt wird, dem ein Umschalter nachgeordnet ist. Diese Vorrichtung enthält ein Polarisationsfilter und eine Flüssigkristallzelle als umschaltbaren Polarisator. Ein Taktgeber schaltet die Flüssigkristallzelle und den Umschalter synchron um. Aus den Ausgangssignalen des Umschalters wird der Quotient gebildet und weiter verarbeitet (DE-OS 27 34 391).

Der Erfindung liegt die Aufgabe zugrunde, diese bekannte Vorrichtung weiter zu verbessern und zugleich ein Verfahren und eine Vorrichtung zur Messung der Konzentration optisch aktiver Substanzen anzugeben, die eine Miniaturisierung und damit eine Implantierung in einem lebenden Körper ermöglichen.

Die Erfindung beruht nun auf der Erkenntnis, daß durch Takten der Strahlungsquelle und Integration der Signalwerte über alternierende Zeitintervalle vor der Quotientenbildung das Signal-Rauschverhältnis dieser bekannten Vorrichtung verbessert werden kann und sie besteht in den kennzeichnenden Merkmalen des Anspruchs 1. Durch die getaktet integrierende Signalverarbeitung in Verbindung mit dem Modulator erhält man ein Auflösungsvermögen für einen Drehwinkel $\alpha_M$ der Polarisationsebene von 5 x $(10^{-4})°$, was beispielsweise bei einer Küvettenlänge von 2,7 cm ein Auflösungsvermögen für eine Glucosekonzentration C von 34 mg/l oder, bezogen auf den physiologischen Normalwert $C_O$ = 800 mg/l, ein relatives Auflösungsvermögen von 4 % ergibt.

Weitere besonders vorteilhafte Ausgestaltungen ergeben sich nach den abhängigen Ansprüchen.

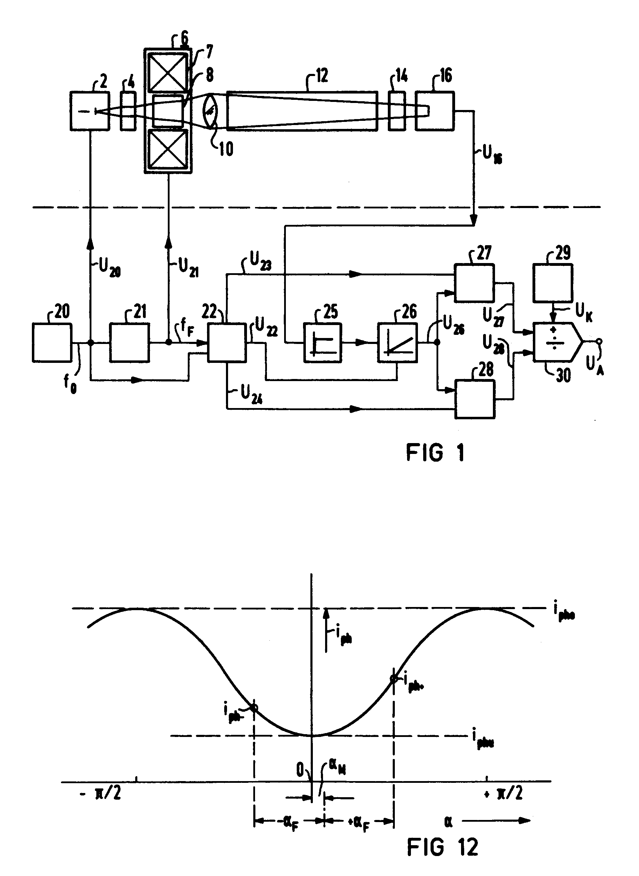

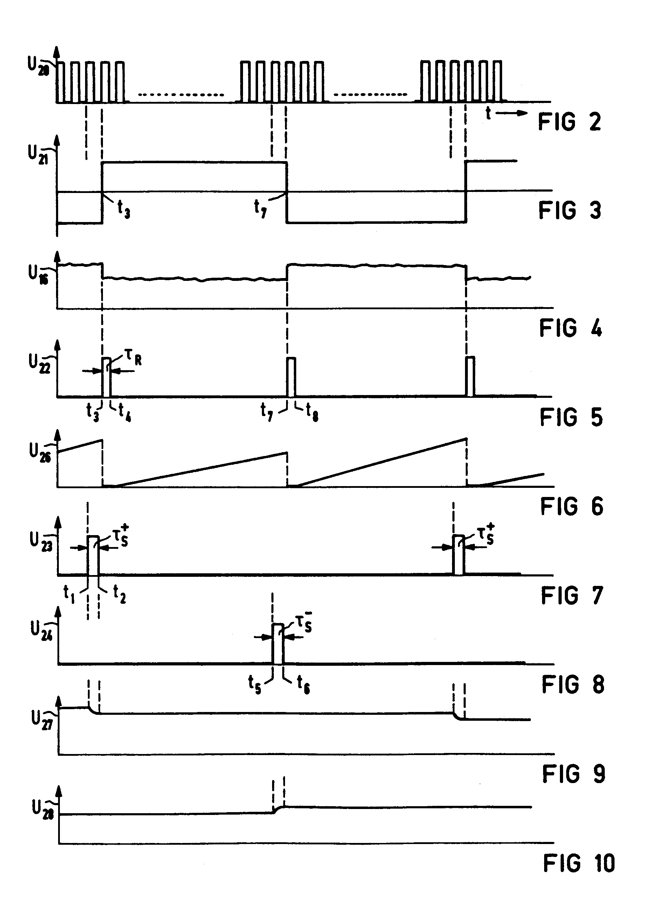

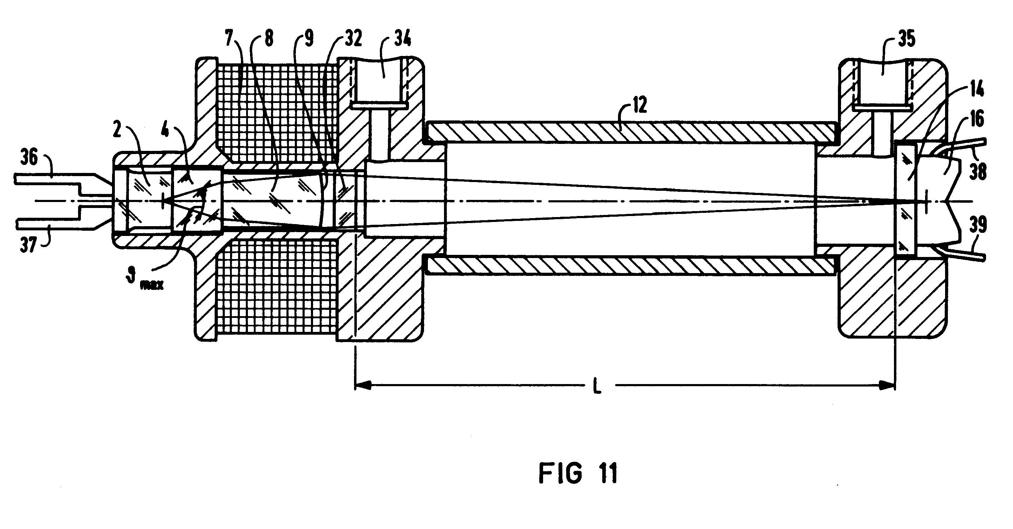

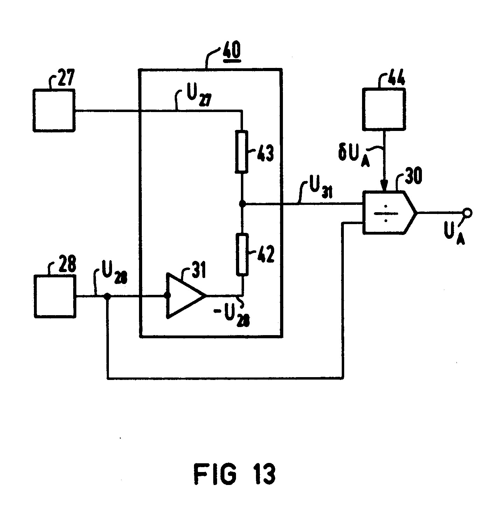

Zur weiteren Erläuterung der Erfindung wird auf die Zeichnung Bezug genommen, in deren Figur 1 als Ausführungsbeispiel der Aufbau und die Schaltung einer Anordnung zur Durchführung des Verfahrens schematisch veranschaulicht sind. Diagramme gemäß den Figuren 2 bis 10 dienen zur Erläuterung der Wirkungsweise der Anordnung nach Figur 1. In Figur 11 ist eine bevorzugte Ausführungsform eines Polarimeters dargestellt. Figur 12 zeigt den Verlauf des Fotostromes in Abhängigkeit vom zu messenden Winkel und vom Modulationswinkel. In Figur 13 ist eine abweichende Ausführungsform der Verarbeitung der beiden Speicherinhalte dargestellt.

In einer Anordnung zur Durchführung des Verfahrens gemäß Figur 1, die vorzugsweise zur Bestimmung der Glucosekonzentration in Körperflüssigkeiten, insbesondere in Blutserum, verwendet werden kann, ist eine Lichtquelle, die vorzugsweise eine Lumineszenzdiode LED sein kann, mit 2, ein Polarisator mit 4 und ein Modulator mit 6 bezeichnet, der vorzugsweise ein Faraday-Modulator sein kann. Dieser Modulator 6 enthält in der Ausführung als Faraday-Modulator eine Spule 7 und einen Kristall 8. Eine Linse, die beispielsweise aus Glas oder Kunststoff bestehen kann, ist mit 10, eine Meßküvette für eine flüssige zu messende Substanz mit 12, ein Analysator mit 14 und ein Detektor mit 16 bezeichnet. In der Meßküvette 12 wird die Schwingungsebene des durch den Polarisator 4 wenigstens annähernd linear polarisierten Lichtes durch eine in der Figur zur Vereinfachung nicht dargestellte optisch aktive Substanz um einen Winkel $\alpha_M$ gedreht. Der Analysator 14 wird bei Abwesenheit der optisch aktiven Substanz in der Meßküvette 12 und bei abgeschaltetem Modulator 6 auf ein Durchlässigkeitsminimum justiert.

Ein Oszillator 20 steuert mit seinem Ausgangssignal $U_{20}$, welches die Frequenz $f_O$ hat, die Lichtquelle 2 sowie einen nachgeschalteten Frequenzteiler 21 und eine Zählschaltung 22. Mit der Modulationsfrequenz $f_F$ des Ausgangssignals $U_{21}$ werden der Modulator 6 und die Zählschaltung 22 gesteuert. Das Ausgangssignal $U_{22}$ der Zählschaltung 22 wird als logisches Steuersignal einem Integrator 26 vorgegeben, dessen Eingang das Ausgangssignal $U_{16}$ des Detektors 16 vorzugsweise über einen Verstärker 25 zugeführt wird. Die beiden weiteren Ausgangssignale $U_{23}$ und $U_{24}$ werden jeweils einem Eingang eines Speichers 27 bzw. 28 vorgegeben, dessen zweitem Eingang das Ausgangssignal $U_{26}$ des Integrators 26 zugeführt wird. Als Speicher 27 und 28 können vorzugsweise Sample-and-Hold-Speicher vorgesehen sein. Die Ausgangssignale $U_{27}$ und $U_{28}$ der Speicher 27 und 28 werden einem Dividierer 30 mit zusätzlichem Additionseingang vorgegeben. Dieser zusätzliche Eingang erhält eine konstante Spannung $U_K$ von einer Konstantspannungsquelle 29.

In der Ausführungsform gemäß Figur 1 ist zur Aufnahme einer flüssigen zu messenden Substanz die Meßküvette 12 vorgesehen. Die Anordnung gemäß der Erfindung kann jedoch auch zur Messung einer festen Substanz verwendet werden, beispielsweise einer organischen Substanz mit asymmetrischer Kohlenstoffbindung, oder allgemein eines optisch aktiven Kristalls, wie z.B. Quarz, Zinnober oder eines der schwefelsauren Doppelsalze der Alkalimetalle. In diesem Fall wird anstelle der Meßküvette 12 die feste Substanz eingebracht.

Gemäß dem Diagramm der Figur 2, in dem die Ausgangsspanung $U_{20}$ des Oszillators 20 in Abhängigkeit von der Zeit t aufgetragen ist, liefert der Oszillator 20 eine konstante Ausgangsfrequenz $f_O$. Der Frequenzteiler 21 liefert gemäß dem Diagramm der Figur 3 ein Ausgangssignal $U_{21}$ mit der Modulationsfrequenz $f_F$, die vorzugsweise wenigstens eine, insbesondere etwa zwei Größenordnungen niedriger ist als die Oszillatorfrequenz $f_O$. Der Photostrom $U_{16}$ des Detektors 16 ist dem Diagramm der Figur 4 zu entnehmen. Angedeutet sind das Rauschen des Photostromes bzw. der ihm proportionalen Spannung $U_{16}$ sowie seine unterschiedlichen mittleren Amplituden in den beiden Phasen des Faraday-Stromes. Bei verschwindendem Drehwinkel $\alpha_M = 0$ innerhalb der Meßküvette 12 sind beide Amplituden gleich groß. Nach der Verstärkung des Photostromes $U_{16}$ und Integration erhält man gemäß dem Diagramm der Figur 6 ein Ausgangssignal $U_{26}$ des Integrators 26, das gemäß Figur 5 zur Zeit $t_3$ und $t_7$ durch die Rückstellimpulse $U_{22}$ mit der Länge $\tau_R$ von $t_3$ bis $t_4$ und von $t_7$ bis $t_8$ zurückgestellt wird. Zur Zeit $t_1$ bis $t_2$ und $t_5$ bis $t_6$ steuern die Steuerimpulse $U_{23}$ und $U_{24}$ gemäß den Diagrammen der Figuren 7 bzw. 8 die Speicher 27 und 28 alternierend in den Sampling-Zustand. Gemäß den Diagrammen der Figuren 9 und 10 ändern sich die Ausgangsspannungen $U_{27}$ und $U_{28}$ der Speicher 27 bzw. 28 nur jeweils während der zugehörigen Sample-Impulse $\tau_S +$ und $\tau_S -$. Ihre Differenz bzw. ihr Quotient Q liefert nach Subtraktion der konstanten Spannung $U_K$ das Meßsignal $U_A$, das dem Drehwinkel $\alpha_M$ der Polarisationsebene in der Meßküvette 12 und somit der Konzentration der in ihr enthaltenen optisch aktiven Substanz proportional ist. Mit dieser Ausführungsform der Meßanordnung erhält man ein Auflösungsvermögen für den Drehwinkel der Polarisationsebene von

$$\Delta \alpha_M = 5 \times (10^{-4})° \ .$$

In der besonderen Ausführungsform eines Polarimeters für eine implantierbare Glucose-Meßanordnung gemäß Figur 11 sind die Lichtquelle 2, der Polarisator 4 und der Faraday-Kristall 8 miteinander verkittet. Die Abbildung der strahlenden Kristallfläche der Lichtquelle 2 auf den Detektor 16 erfolgt ausschließlich durch die Linsenwirkung der mit 9 bezeichneten konvexen Rückseite des Faraday-Kristalls 8, die vorzugsweise durch eine $\lambda/4$-Schicht aus Siliziumoxid SiO oder einer höher oxidierten Mischform $SiO_x$, mit $1 < x \leq 2$, entspiegelt sein kann. Die Meßküvette 12 mit beispielsweise einer Länge L = 27 mm ist auf der Eintrittsseite durch ein Fenster 32, vorzugsweise ein Neutralglasfenster, und auf der Austrittsseite durch den Analysator 14 abgeschlossen. Zwei Bohrungen 34 und 35 dienen zur Durchströmung mit der Meßflüssigkeit, insbesondere mit einem filtrierten Blutserum. Elektrische Anschlüsse für die Lichtquelle 2 sind mit 36

EP 0 351 659 B1

und 37 und für den Detektor 16 mit 38 und 39 bezeichnet. Die Anschlüsse für die Faraday-Spule 7 sind in der Figur zur Vereinfachung nicht dargestellt. Das angedeutete Strahlenbündel ist durch den maximal nutzbaren Öffnungswinkel 2 $\vartheta_{max}$ der von der Lichtquelle 2 emittierten Strahlung begrenzt.

In dieser Ausführungsform eines Polarimeters gemäß Figur 11, bei der die Lichtquelle 2, der Polarisator 4 und der Faraday-Kristall 8 durch Immersionsschichten miteinander verbunden bzw. verkittet sind, erhält man praktisch keine Reflexionsverluste und es ergibt sich eine mechanisch stabile Montage.

Als Lichtquelle 2 kann vorzugsweise eine Lumineszenzdiode LED vorgesehen sein, die eine monochromatische Strahlung liefert und verhältnismäßig kleine, billig, stabil und hochfrequent taktbar ist.

Mit einer Abbildung der Lichtquelle 2 mit Linsen auf den Detektor 16 erhält man eine maximale Amplitude des Photostromes und somit ein günstiges Signal/Rauschverhältnis. Durch die konvexe Rückfläche 9 des Faraday-Kristalls 8 ergibt sich eine Wirkung als Immersionslinse in bezug auf die Lichtquelle 2, und der hohe Brechungsindex, beispielsweise n = 3,4 für Galliumphosphid GaP bei einer Wellenlänge $\lambda$ = 575 nm (gelb) kann ausgenutzt werden. Dadurch können Linsen zur Abbildung der Lichtquelle 2 auf den Detektor ganz oder wenigstens teilweise eingespart werden. Als Lichtquelle 2 ist auch eine Halbleiter-Laserdiode wegen ihrer hohen Strahlungsleistung und der damit erreichbaren höchsten Meßempfindlichkeit geeignet.

Als optisches Medium für den Faraday-Kristall 8 des Modulators 6 ist vorzugsweise eine $A_{III}B_V$-Verbindung geeignet. Diese Verbindungen haben eine große Verdet'sche Konstante V, d.h. man erhält einen starken Faraday-Effekt bei einem verhältnismäßig kleinen Magnetfeld in der Faraday-Spule 7. Insbesondere geeignet ist Gallium-Phosphid mit einer Verdet-Konstante V = 4,5 x $10^3$ min/(T x cm) bei einer Wellenlänge $\lambda$ = 665 nm bis V = 6,7 x $10^3$ min/(T x cm) bei einer Wellenlänge $\lambda$ = 560 nm. Als Detektor 16 ist vorzugsweise eine Silicium-Photodiode oder auch ein Silicium-Photoelement geeignet.

Als optisches Medium für den Faraday-Kristall 8 des Modulators 6 ist ferner ein Granat geeignet, der in großen Kristallen nach dem Czochralski-Verfahren rein herstellbar ist und eine hohe Verdet'sche Konstante hat. Vorzugsweise geeignet ist ein Granat auf der Basis von Gadolinium-Gallium; diese Granate sind im gesamten sichtbaren Spektralbereich transparent. Gut geeignet sind auch Eisen-Granate vom Typ $M_3Fe_5O_{12}$, wobei M eine der Seltenen Erden, insbesondere Ytterbium, Thulium, Yttrium, Erbium oder Holmium ist. Diese Seltenen Erden haben eine sehr hohe Verdet'sche Konstante unterhalb der magnetischen Sättigung, beispielsweise V = 4 x $10^4$ min/(T x cm) für Yttrium-Eisen-Granat $Y_3Fe_5O_{12}$ bei einer Wellenlänge von $\lambda$ = 1 $\mu$m. Damit sind sehr kurze Faraday-Kristalle 8 mit weniger als 1 mm Länge anwendbar, und die sie umschließende Spule 7 kann sehr klein gemacht werden, was wiederum die zur Magnetfeld-Erzeugung erforderliche elektrische Energie vermindert. Dies ist besonders wichtig für den implantablen Glucosesensor, da hier nur sehr leistungsschwache Stromversorgungen eingesetzt werden können.

In Verbindung mit einem Eisen-Granat kann als Lichtquelle vorzugsweise eine Infrarot-Lumineszenzdiode aus Gallium-Indium-Arsenid-Phosphid $Ga_xIn_{1-x}As_yP_{1-y}$ verwendet werden, mit einer Emissionswellenlänge im Bereich 0,9 $\mu$m ≤ $\lambda$ ≤ 1,4 $\mu$m, bevorzugt im Bereich 1,0 $\mu$m ≤ $\lambda$ ≤ 1,11 $\mu$m. In diesem Bereich ist nämlich die Eindringtiefe der Strahlung in Wasser größer als 60 mm, so daß Küvettenlängen dieser Größenordnung einsetzbar sind und damit entsprechend kleine Konzentrationen der optisch aktiven Substanz, insbesondere Glucose, meßbar sind. In Verbindung mit einem Eisen-Granat als Kristall 8 kann als Detektor 16 vorzugsweise eine Germanium-Photodiode oder ein Germanium-Photoelement, insbesondere für Wellenlängen im Bereich 1,0 $\mu$m ≤ $\lambda$ ≤ 1,7 $\mu$m, eingesetzt werden.

Die Steuerspannung $U_{21}$ mit der Modulationsfrequenz $f_F$ für die Faraday-Spule 7 ist eine symmetrische Rechteckspannung, wie dem Diagramm der Figur 2 zu entnehmen ist. Mit dieser Steuerspannung erhält man das größtmögliche Detektorsignal $U_{16}$ bei vorgegebener Amplitude des Spulenstromes. Dabei wird die Oszillatorfrequenz $f_O$ nähernd 100 x $f_F$ und die Modulationsfrequenz $f_F \lessapprox$ 500 Hz gewählt.

Die Oszillatorfrequenz $f_O$, die zugleich die Taktfrequenz $f_L$ der Lichtquelle 2 darstellt, kann vorzugsweise wesentlich größer als die Modulationsfrequenz $f_F$ gewählt werden, wie es den Diagrammen der Figuren 2 und 3 zu entnehmen ist. Die Modulationsfrequenz $f_F$ wird vorzugsweise im Bereich 50 Hz ≤ $f_F$ ≤ 500 Hz gewählt. Mit der Taktfrequenz $f_L$ erhält man in Verbindung mit einer Lumineszenzdiode als Lichtquelle 2 durch Ausnutzung der nichtlinearen Emission/Betriebsstrom-Charakteristik der Lumineszenzdiode eine erhöhte Lichtausbeute, insbesondere im sichtbaren Spektralbereich. Damit erhält man wiederum ein ensprechend verbessertes Signal/ Rausch-Verhältnis und somit eine entsprechend höhere Meßempfindlichkeit. Mit einer Oszillatorfrequenz $f_O$ als Taktfrequenz $f_L$ der Lichtquelle 2 erhält man ohne zusätzlichen Schaltungsaufwand eine genau gleiche Anzahl von Strahlungsimpulsen der Lichtquelle 2 in jeder Integrationsperiode der Dauer 1/(2 x $f_F$) und somit eine hohe Stabilität des Meßsignals.

Die Startzeitpunkte $t_1$ und $t_5$ der Zeitintervalle $\tau_{s+}$ und $\tau_{s-}$ für die Sample-Impulse der Sample-and-Hold-Speicher 27 und 28 sowie die Startzeitpunkte $t_3$ und $t_7$ der Zeitintervalle $\tau_R$ für die Rückstellung des

4

Integrators 26 erfolgen vorzugsweise durch Abzählen der $f_O$- bzw. $f_L$-Perioden während jeweils einer $f_F$-Periode. Dadurch ergeben sich sehr genau gleiche Integrationszeiten für jeden der in die beiden Speicher 27 und 28 einzuspeichernden Integralwerte.

Durch die Verfahrensmerkmale gemäß der Erfindung erhält man ein rausch- und driftarmes Ausgangssignal $U_A = (U_{27}/U_{28}) \times U_N - U_K = Q - U_K$, das durch entsprechende Wahl der konstanten Spannung $U_K$ bei Null- bzw. Untergrund-Drehwinkel der Polarisationsebene in der Meßküvette 12 zu Null gemacht werden kann und somit als Maß für die gesuchte Konzentration der optisch aktiven Substanz dem durch diese Substanz bewirkten Drehwinkel $\Delta \alpha_M$ der Polarisationsebene proportional ist, als $U_A \sim \Delta \alpha_M$. $U_N$ ist die konstante interne Normierungsspannung des Dividierers 30.

Primäres Meßsignal ist der im Detektor 16 erzeugte Photostrom $i_{ph}$. Die Hauptdurchlaßrichtungen vom Polarisator 4 und Analysator 14 sind senkrecht zueinander, d.h. auf minimale Durchlässigkeit, eingestellt. Die dieser Situation entsprechende Schwingungsrichtung der auf den Analysator 14 treffenden linear polarisierten Strahlung wird durch den Drehwinkel $\alpha = 0$ der Schwingungsebene gekennzeichnet. Die Polarisationsebene der den Polarisator 4 verlassenden Strahlung wird durch den die Faraday-Spule 7 durchfließenden Wechselstrom der Frequenz $f_F$ alternierend um maximal die dem Betrage nach gleichen Winkel $\pm \alpha_F$ gedreht. In der Küvette 12 bewirkt die zu messende optisch aktive Substanz eine weitere Drehung um den der Konzentration C dieser Substanz proportionalen Winkel $\alpha_M$. Dadurch oszilliert der Photostrom mit der Frequenz $f_F$ zwischen einem vom Polarisationsgrad abhängigen Minimalwert und den alternierend erzeugten Extremwerten

$$i_{ph\pm} = i_o \times \sin^2(\pm\alpha_F + \alpha_M) + i_u \times \cos^2(\pm\alpha_F + \alpha_M)$$

$$= i_u + (i_o - i_u) \times \sin^2(\pm\alpha_F + \alpha_M). \tag{1}$$

Bei rechteckförmigen Wechselstrom in der Faraday-Spule 7 werden nur die beiden Werte $i_{ph+}$ und $i_{ph-}$ alternierend erzeugt.

Im Idealfall vollständig linearer Polarisation der den Analysator 14 erreichenden Strahlung sowie Polarisationsgrad 1 des Analysators 14 ist die "Depolarisationskomponente" $i_u = $ Null. Bei abgeschaltetem Faraday-Modulator 6 und ohne optisch aktive Substanz in der Meßküvette 12, d.h. bei $\alpha_F = \alpha_M = 0$, verschwindet dann der Photostrom $i_{ph}$.

Für den allgemeinen Fall unvollständiger Polarisation $0 < i_u < i_o$ ist die Bildung des Photostromes $U_{16}$ mit den unterschiedlichen Amplituden $i_{ph+}$ und $i_{ph-}$ in Figur 12 dargestellt. Die Depolarisationskomponente $i_u$ ist dabei übertrieben groß gezeichnet ($i_u = i_o/4$). Mit handelsüblichen Polarisatoren läßt sich $i_u < i_o/100$ erzielen. Führt man als Maß für den Polarisationsgrad p der den Detektor 16 erreichenden Strahlung das Verhältnis

$$\epsilon = i_u/i_o = (1-p) \tag{2}$$

ein, dann nimmt die Gleichung (1) für die beiden Photoströme, die bei Rechteckmodulation durch die Faraday-Spule 7 alternierend erzeugt werden, die folgende Form an:

$$i_{ph\pm} = i_o[\epsilon + (1-\epsilon) \times \sin^2(\pm\alpha_F + \alpha_M)]. \tag{3}$$

Der Photostrom $i_{ph}$ wird in dem Verstärker 25 zu der Spannung $U_v = v \times i_{ph}$ verstärkt und anschließend gemäß der Erfindung im Integrator 26 alternierend zu den beiden Spannungen

$$U_{I\pm} = \frac{1}{R_J \times C_J} \times \int_0^{\tau_J} U_{v\pm} \, dt$$

$$= \frac{\tau_J}{R_J \times C_J} \times \overline{U_{v\pm}} \tag{4}$$

aufintegriert, mit $R_J \times C_J$ als Zeitglied des Integrators 26. $\tau_J$ ist die wirksame Integrationszeit. Sie ist durch den Oszillator 20, den Frequenzteiler 21 und die Zählschaltung 22 festgelegt und ist gleich der Zeitdifferenz $t_5 - t_4$ zwischen dem Ende eines Rückstellimpulses $U_{22}$ zur Zeit $t_4$ und dem nächstfolgenden Speicherimpuls $U_{24}$ zur Zeit $t_5$. Der Integrator 26 wirkt in bekannter Weise als Mittelwertverstärker mit einstellbarer Verstärkung.

Die alternierend erzeugten Integralwerte $U_{I\pm}$ der Spannung $U_{26}$ werden in den durch die Spannungsimpulse $U_{23}$ und $U_{24}$ definierten Zeitintervallen $\tau_S+$ und $\tau_S-$ getrennt in die Speicher 27 und 28 eingespeichert. Aus den jeweils aktuellen Speicherinhalten und der additiven Konstantspannung $U_K$ der Konstantspannungsquelle 29 wird schließlich im Dividierer 30 die Ausgangsspannung $U_A$ erzeugt und man erhält

$$U_A = \frac{U_{I+}}{U_{I-}} \times U_N - U_K$$

$$= \frac{\overline{I}_{ph+}}{\overline{I}_{ph-}} \times U_N - U_K \tag{5}$$

$$= \frac{U_{27}}{U_{28}} \times U_N - U_K \; .$$

Die Querstriche über $i_{ph+}$ und $i_{ph-}$ bedeuten, daß die beiden Photoströme jeweils über eine zugehörige Periode $\tau_J$ gemittelt sind. $U_N$ ist die bereits erwähnte interne Normierungsspannung des Dividierers 30, beispielsweise 10 V. Wählt man die Konstantspannung $U_K$ gleich der internen Normierungsspannung $U_N$, dann erhält man

$$U_A = \left( \frac{\overline{i}_{ph+}}{\overline{i}_{ph-}} - 1 \right) \times U_N . \tag{6}$$

Mit dem nach der Gleichung 3 berechneten Photostrom bzw. der ihm proportionalen Spannung $U_{16}$ erhält man das auf die Spannung $U_N$ normierte Ausgangssignal zu

$$y = \frac{U_A}{U_N} = \frac{(1-\epsilon) \times \sin 2\alpha_M \times \sin 2\alpha_F}{\epsilon + (1-\epsilon) \times \sin^2(\alpha_M - \alpha_F)} \; . \tag{7}$$

Entsprechend der durch die Gleichung 4 beschriebenen Integration sind die Größen $\alpha_M$, $\alpha_F$ und $\epsilon$ auf der rechten Seite der Gleichung 7 als Mittelwerte über jeweils eine Integrationsperiode $\tau_J$ zu interpretieren. Damit sind auch die Ausgangsspannung $U_A$ und das normierte Ausgangssignal y Mittelwerte mit entsprechend vermindertem Rauschanteil. Zur weiteren Verminderung der Rauschbandbreite wird der Dividierer 30 vorzugsweise mit einer Zeitkonstante $\tau_Q > 1/f_F$ gewählt.

Für den bei der Glucose-Konzentrationsbestimmung besonders wichtigen Fall kleiner Drehung der Polarisationsebene $\alpha_M \ll \alpha_F$ und außerdem kleiner Faraday-Drehung $\sin \alpha_F \ll 1$, beispielsweise $\alpha_F = 2°$, $\sin \alpha_F = 0,035$, vereinfacht sich die Gleichung 7 zu

$$y = 4 \times \frac{\alpha_M}{\alpha_F} \times \left( 1 - \frac{1}{1 + (\frac{1}{\epsilon} - 1) \times \alpha_F^2} \right) \; . \tag{8}$$

EP 0 351 659 B1

Ist außerdem die Strahlung hinreichend gut linear polarisiert, so daß insbesondere

$$\mathcal{E} = i_u/i_o \ll \frac{1}{1+1/\alpha_F^2} \, , \qquad (9)$$

dann ist der Bruchterm in Gleichung (8) zu vernachlässigen und das Ausgangssignal ist dem Drehwinkel $\alpha_M$ der Polarisationsebene und damit auch der Konzentration C der zu bestimmenden optisch aktiven Substanz in der Küvette 12 proportional:

$$y = 4 \times \alpha_M/\alpha_F, \qquad (10)$$

beziehungsweise

$$U_a = 4 \times (\alpha_M/\alpha_F) \times U_N \, . \qquad (11)$$

Entsprechend den verschiedenen algebraischen Darstellungsmöglichkeiten von Gleichung (5), beispielsweise in der Form

$$U_A = (U_{27}-U_{28}) \times \frac{U_N}{U_{28}} + U_N - U_K$$

$$\qquad\qquad\qquad\qquad\qquad\qquad (5a)$$

$$\quad = (U_{27}-U_{28}) \times \frac{U_N}{U_{28}} + \delta U_A$$

kann die Ausgangsspannung $U_A$ aus den beiden Ausgangssignalen $U_{27}$ und $U_{28}$ in bekannter Weise auch durch eine Subtraktion und anschließende Division ermittelt werden. Die genaue Einstellung des Nullpunktes zur Kompensation kleiner Abweichungen zwischen den Photoströmen $\bar{i}_{ph+}$ und $\bar{i}_{ph-}$ bzw. zwischen den Ausgangssignalen $U_{27}$ und $U_{28}$ bei der Justierung des Analysators 14 auf minimale Durchlässigkeit, erfordert dann nur die Addition einer kleinen Offsetspannung $\delta U_A = U_N-U_K$. Daher kann die Ausgangsbeschaltung der beiden Sample-and-Hold-Speicher 27 und 28 in Figur 1 beispielsweise auch durch ein gleichwertiges Schaltschema nach Figur 13 dargestellt werden. In dieser Schaltung wird im Subtrahierer 40, der beispielsweise aus dem Invertierer 31 und den beiden Widerständen 42 und 43 bestehen kann, die Differenzspannung $U_{31} = (U_{27}-U_{28})/2$ gebildet. Anschließend wird - unter Berücksichtigung des Faktors 2 - im Dividierer 30 durch das Ausgangssignal $U_{28}$ dividiert. Die Konstantspannungsquelle 44 hat lediglich die geringe Offsetspannung $\delta U_A$ zu liefern, welche bei idealer Justierung Null ist.

Im Ausführungsbeispiel gemäß Figur 1 ist eine analoge Signalverarbeitung vorgesehen. Die Funktionselemente im unteren Teil der Figur 1 können jedoch beispielsweise teilweise auch durch einen Mikroprozessor ersetzt werden, der die Funktion des Oszillators 20, des Frequenzteilers 21, der Zählschaltung 22 sowie des Integrators 26 und der Sample-and-Hold-Speicher 27 und 28 und des Dividierers 30 mit der Konstantspannungsquelle 29 übernimmt.

Im Ausführungsbeispiel ist als Modulator 6 ein Faraday-Modulator vorgesehen. Unter Umständen kann es zweckmäßig sein, einen Kerr-Modulator zu verwenden, bei dem durch eine elektrische Feldstärke eine künstliche Doppelbrechung und damit eine Drehung der Polarisationsebene erzeugt wird.

**Patentansprüche**

**1.** Verfahren zur Messung der Konzentration optisch aktiver Substanzen durch Polarisationsmessung mit polarisiertem Licht einer Lichtquelle, das über einen Modulator, der mit einer vorbestimmten Modulationsfrequenz betrieben wird, sowie eine zu messende Substanz und einen Analysator einem Detektor zugeführt wird, **gekennzeichnet** durch folgende Merkmale:
    a) die Modulationsfrequenz $f_F$ wird durch Frequenzteilung einer Oszillatorfrequenz $f_O$ erzeugt;
    b) die Lichtquelle (2) wird mit einer Taktfrequenz $f_L$ getaktet, die wesentlich höher als die Modulationsfrequenz $f_F$ gewählt wird;

c) das Ausgangssignal( $U_{16}$) des Detektors (16) wird während jeder Halbperiode der Modulationsfrequenz $f_F$ abwechselndzu jeweils einem Spannungswert ($U_I$+ bzw. $U_I$-) aufintegriert;

d) diese Spannungswerte( $U_I$+ und $U_I$-) werden gespeichert und dann wird der Quotient (Q) dieser gespeicherten Spannungswerte gebildet;

e) anschließend wird eine einstellbare konstante Spannung ($U_K$) vom Quotienten (Q) subtrahiert.

2. Verfahren nach Anspruch 1, **dadurch gekennzeichnet,** daß die Oszillatorfrequenz $f_O \approx 100 \times f_F$ gewählt wird und die Modulationsfrequenz $f_F$ zwischen 50 und 500 Hz liegt.

3. Verfahren nach Anspruch 1, **dadurch gekennzeichnet,** daß als Taktfrequenz $f_L$ der Lichtquelle (2) die Oszillatorfrequenz $f_O$ gewählt wird.

4. Anordnung zur Durchführung des Verfahrens nach einem der Ansprüche 1 bis 3, **dadurch gekennzeichnet,**
   - daß dem Detektor (16) ein Integrator (26) nachgeschaltet ist,
   - daß die Ausgangssignale ($U_{26}$) dieses Integrators (26) jeweils abwechselnd in einen von zwei Speichern (27 bzw. 28) eingespeichert werden,
   - daß die Inhalte der beiden Speicher (27 bzw. 28) einem Dividierer (30) vorgegeben sind, dessen zusätzlicher Additionseingang an eine Konstantspannungsquelle (29) angeschlossen ist,
   - daß ein Oszillator (20) mit einer Oszillatorfrequenz ($f_O$) vorgesehen ist,
   - daß diese Oszillatorfrequenz ($f_O$) als Taktftrequenz ($f_L$) für die Lichtquelle (2) vorgesehen ist und zugleich einen Frequenzteiler (21) steuert,
   - daß dieser Frequenzteiler (21) eine Modulationsfrequenz ($f_F$) erzeugt, welche sowohl einen Modulator (6) als auch eine Zählschaltung (22) steuert, deren zweiter Eingang an den Ausgang des Oszillators (20) angeschlossen ist,
   - daß die Zählschaltung (22) Zeitglieder zur Steuerung von Pulsspannungen ($U_{22}$, $U_{23}$, $U_{24}$) enthält, die dem Integrator (26) sowie den Speichern (27 bzw. 28) als logische Eingangssignale vorgegeben sind (Figur 1).

5. Anordnung nach Anspruch 4, **dadurch gekennzeichnet,** daß als Speicher (27, 28) Sample-and-Hold-Speicher vorgesehen sind, denen ein Subtrahierer (40) nachgeschaltet ist, dem der Dividierer (30) nachgeschaltet ist (Figur 13).

6. Anordnung nach Anspruch 4, **dadurch gekennzeichnet,** daß der Modulator (6) ein Faraday-Modulator ist.

7. Anordnung nach einem der Ansprüche 4 bis 6, **dadurch gekennzeichnet,** daß zur Bildung des Quotienten (Q) ein Dividierer 30 mit zusätzlichem Additionseingang vorgesehen ist, dessen Zeitkonstante $\tau_Q > 1/f_F$ ist.

8. Anordnung nach einem der Ansprüche 4 bis 7, **dadurch gekennzeichnet,** daß als Lichtquelle (2) eine Lumineszenzdiode LED vorgesehen ist.

9. Anordnung nach einem der Ansprüche 4 bis 8, **dadurch gekennzeichnet,** daß als Lichtquelle (2) eine Halbleiter-Laserdiode vorgesehen ist.

10. Anordnung nach einem der Ansprüche 6 bis 9, **dadurch gekennzeichnet,** daß der Modulator (6) einen Faraday-Kristall (8) aus einer $A_{III}B_V$-Verbindung enthält.

11. Anordnung nach Anspruch 10, **dadurch gekennzeichnet,** daß ein Faraday-Kristall (8) aus Gallium-Phosphid GaP vorgesehen ist.

12. Anordnung nach Anspruch 10, **dadurch gekennzeichnet,** daß ein Faraday-Kristall (8) aus Gallium-Arsenid GaAs vorgesehen ist.

13. Anordnung nach einem der Ansprüche 6 bis 9, **dadurch gekennzeichnet,** daß der Modulator (6) einen Faraday-Kristall (8) aus einer $A_{1III}xA_{2III(1-x)}B_{1Vy}B_{2V(1-y)}$-Verbindung enthält, bei der $0 \leq (x, y) \leq 1$ ist.

**14.** Anordnung nach einem der Ansprüche 6 bis 13, **dadurch gekennzeichnet,** daß die Lichtquelle (2), der Polarisator (4) und der Faraday-Kristall (8) durch Immersionsschichten miteinander verbunden sind.

**15.** Anordnung nach einem der Ansprüche 4 bis 14, **dadurch gekennzeichnet,** daß zur Abbildung der Lichtquelle (2) auf den Detektor (16) Linsen vorgesehen sind.

**16.** Anordnung nach einem der Ansprüche 6 bis 15, **dadurch gekennzeichnet,** daß ein Faraday-Kristall (8) mit konvexer Rückfläche (9) vorgesehen ist.

**17.** Anordnung nach einem der Ansprüche 6 bis 16, **dadurch gekennzeichnet,** daß als Faraday-Kristall (8) ein Granat vorgesehen ist.

**18.** Anordnung nach Anspruch 17, **gekennzeichnet** durch einen Granat auf der Basis von Gadolinium-Gallium.

**19.** Anordnung nach Anspruch 17, **gekennzeichnet** durch einen Eisen-Granat vom Typ $M_3Fe_5O_{12}$, bei dem M eine der Seltenen Erden, insbesondere Ytterbium Yb oder Thulium Tm oder Yttrium Y oder Erbium Er oder Holmium Ho ist.

**20.** Anordnung nach Anspruch 19, **dadurch gekennzeichnet,** daß als Lichtquelle (2) eine Infrarot-Lumineszenzdiode aus $Ga_xIn_{1-x}As_yP_{1-y}$ vorgesehen ist mit einer Emmisionswellenlänge $\lambda$ im Bereich 0,9 $\mu m \leq \lambda \leq 1,4\ \mu m$.

**21.** Anordnung nach Anspruch 20, **gekennzeichnet** durch eine Emissionswellenlänge $\lambda$ im Bereich 1 $\mu m \leq \lambda \leq 1,11\ \mu m$.

**22.** Anordnung nach Anspruch 20 oder 21, **dadurch gekennzeichnet,** daß der Detektor (16) aus einer Germanium-Photodiode oder einem Germanium-Photoelement besteht.

**23.** Anordnung nach einem der Ansprüche 4 bis 22, **dadurch gekennzeichnet,** daß als Steuerspannung ($U_{21}$) mit der Modulationsfrequenz ($f_F$) für den Modulator (6) eine symmetrische Rechtckspannung vorgesehen ist.

**Claims**

**1.** Method for measuring the concentration of optically active substances through polarization measurement with polarized light of a light source, the light being supplied to a detector by way of a modulator which is operated with a predetermined modulation frequency, as well as by way of a substance to be measured and an analyzer, characterized by the following features:
   a) the modulation frequency $f_F$ is generated by frequency division of an oscillator frequency $f_O$;
   b) the light source (2) is clocked by a clock frequency $f_L$ which is selected to be substantially higher than the modulation frequency $f_F$;
   c) the output signal ($U_{16}$) of the detector (16) is integrated during each half period of the modulation frequency $f_F$ alternately to in each case one voltage value ($U_I+$ and $U_I-$);
   d) these voltage values ($U_I+$ and $U_I-$) are stored and the quotient (Q) of these stored voltage values is then formed;
   e) subsequently, an adjustable constant voltage ($U_K$) is subtracted from the quotient (Q).

**2.** Method according to claim 1, characterized in that the oscillator frequency $f_O \approx 100 \times f_F$ is selected and the modulation frequency $f_F$ lies between 50 and 500 Hz.

**3.** Method according to claim 1, characterized in that the oscillator frequency $f_O$ is selected as clock frequency $f_L$ of the light source (2).

**4.** Arrangement for carrying out the method according to one of claims 1 to 3, characterized in that
   - an integrator (26) is connected after the detector (16),
   - the output signals ($U_{26}$) of this integrator (26) are stored in each case alternately into one of two memories (27 and 28),

9

- the contents of the two memories (27 and 28) are specified for a divider (30), the additional addition input of which is connected to a constant-voltage source (29),
- an oscillator (20) with an oscillator frequency ($f_O$) is provided,
- this oscillator frequency ($f_O$) is provided as clock frequency ($f_L$) for the light source (2) and at the same time controls a frequency divider (21),
- this frequency divider (21) generates a modulation frequency ($f_F$) which controls both a modulator (6) and a counting circuit (22), the second input of which is connected to the output of the oscillator (20),
- the counting circuit (22) contains time elements for the control of pulse voltages ($U_{22}$, $U_{23}$, $U_{24}$) which are specified for the integrator (26) and the memories (27 and 28) as logical input signals (Figure 1).

5. Arrangement according to claim 4, characterized in that there are provided as memories (27, 28) sample-and-hold memories, after which there is connected a subtracter (40), after which there is connected the divider (30) (Figure 13).

6. Arrangement according to claim 4, characterized in that the modulator (6) is a Faraday modulator.

7. Arrangement according to one of claims 4 to 6, characterized in that to form the quotient (Q) a divider 30 with additional addition input is provided, the time constant of which is $\tau_Q > 1/f_F$.

8. Arrangement according to one of claims 4 to 7, characterized in that a light-emitting diode LED is provided as light source (2).

9. Arrangement according to one of claims 4 to 8, characterized in that a semiconductor laser diode is provided as light source (2).

10. Arrangement according to one of claims 6 to 9, characterized in that the modulator (6) contains a Faraday crystal (8) consisting of a $A_{III}B_V$ compound.

11. Arrangement according to claim 10, characterized in that a Faraday crystal (8) of gallium phosphide GaP is provided.

12. Arrangement according to claim 10, characterized in that a Faraday crystal (8) of gallium arsenide GaAs is provided.

13. Arrangement according to one of claims 6 to 9, characterized in that the modulator (6) contains a Faraday crystal (8) consisting of a $A_{1IIIx}A_{2III(1-x)}B_{1Vy}B_{2V(1-y)}$ compound, where $0 \leq (x, y) \leq 1$ applies.

14. Arrangement according to one of claims 6 to 13, characterized in that the light source (2), the polarizer (4) and the Faraday crystal (8) are connected to one another by immersion layers.

15. Arrangement according to one of claims 4 to 14, characterized in that lenses are provided to image the light source (2) on to the detector (16).

16. Arrangement according to one of claims 6 to 15, characterized in that a Faraday crystal (8) with convex rear face (9) is provided.

17. Arrangement according to one of claims 6 to 16, characterized in that a garnet is provided as Faraday crystal (8).

18. Arrangement according to claim 17, characterized by a garnet based on gadolinium gallium.

19. Arrangement according to claim 17, characterized by an iron garnet of the $M_3Fe_5O_{12}$ type, where M is one of the rare earths, in particular ytterbium Yb or thulium Tm or yttrium Y or erbium Er or holmium Ho.

20. Arrangement according to claim 19, characterized in that an infrared luminescence diode consisting of

$Ga_xIn_{1-x}As_yP_{1-y}$ is provided as light source (2), with an emission wavelength $\lambda$ in the region of 0.9 $\mu$m $\leq$ $\lambda \leq$ 1.4 $\mu$m.

**21.** Arrangement according to claim 20, characterized by an emission wavelength $\lambda$ in the region of 1 $\mu$m $\leq$ $\lambda \leq$ 1.11 $\mu$m.

**22.** Arrangement according to claim 20 or 21, characterized in that the detector (16) consists of a germanium photodiode or a germanium photo-element.

**23.** Arrangement according to one of claims 4 to 22, characterized in that a symmetrical square-wave voltage is provided as control voltage ($U_{21}$) with the modulation frequency ($f_F$) for the modulator (6).

**Revendications**

**1.** Procédé pour mesurer la concentration de substances actives du point de vue optique, par mesure de polarisation avec une lumière polarisée d'une source de lumière, qui est envoyée à un détecteur par l'intermédiaire d'un modulateur, qui fonctionne avec une fréquence de modulation prédéterminée, ainsi que d'une substance à mesurer et d'un analyseur, caractérisé par les particularités suivantes :
a) la fréquence de modulation $f_F$ est produite par division d'une fréquence $f_O$ d'un oscillateur;
b) la source de lumière (2) est commandée de façon cadencée avec une fréquence de cadence $f_L$, qui est choisie nettement supérieure à la fréquence de modulation $f_F$;
c) le signal de sortie ($U_{16}$) du détecteur (16) est intégré alternativement, pendant chaque alternance de la fréquence de modulation $f_F$, pour former respectivement une valeur de tension ($U_I+$ ou $U_I-$);
d) les valeurs de tension ($U_I+$ et $U_I-$) sont mémorisées et le quotient (Q) de ces valeurs de tension mémorisées est ensuite formé;
e) ensuite, une tension constante réglable ($U_K$) est soustraite du quotient (Q).

**2.** Procédé suivant la revendication 1, caractérisé par le fait que la fréquence de l'oscillateur est choisie égale à $f_O \approx$ 100 x $f_F$ et que la fréquence de modulation $f_F$ se situe entre 50 et 500 Hz.

**3.** Procédé suivant la revendication 1, caractérisé par le fait qu'on choisit comme fréquence de cadence $f_L$ de la source de lumière (2) la fréquence $f_O$ de l'oscillateur.

**4.** Dispositif pour la mise en oeuvre du procédé suivant l'une des revendications 1 à 3, caractérisé par le fait
- qu'un intégrateur (26) est branché en aval du détecteur (16),
- que les signaux de sortie ($U_{26}$) de cet intégrateur (26) sont alternativement mémorisés dans l'une de deux mémoires (27 ou 28),
- que le contenu des deux mémoires (27 ou 28) sont appliqués à un diviseur (30), dont l'entrée d'addition supplémentaire est raccordée à une source de tension constante (29),
- qu'il est prévu un oscillateur (20) possédant une fréquence ($f_O$),
- que cette fréquence ($f_O$) de l'oscillateur est utilisée comme fréquence de cadence ($f_L$) pour la source de lumière (2) et commande simultanément un diviseur de fréquence (21),
- le diviseur de fréquence (21) produit une fréquence de modulation ($f_F$), qui commande aussi bien un modulateur (6) qu'un circuit de comptage (22), dont la seconde entrée est raccordée à la sortie de l'oscillateur (20),
- que le circuit de comptage (22) comporte des circuits de temporisation servant à commander des tensions impulsionnelles ($U_{22}$, $U_{23}$, $U_{24}$) qui sont appliquées a l'intégrateur (26) ainsi qu'aux mémoires (27, 28), en tant que signaux d'entrée logiques.

**5.** Dispositif suivant la revendication 4, caractérisé par le fait qu'il est prévu, comme mémoire (27,28), des mémoires d'échantillonnage et de blocage, en aval desquelles est branché un soustracteur (40), en aval duquel est branché le diviseur (30). (Figure 13).

**6.** Dispositif suivant la revendication 4, caractérisé par le fait que le modulateur (6) est un modulateur de Faraday.

**7.** Dispositif suivant l'une des revendications 4 à 6, caractérisé Par le fait que pour la formation du quotient (Q), il est prévu un diviseur (30) comportant une entrée d'addition supplémentaire et dont la constante de temps est $\tau_Q > 1/f_F$.

8. Dispositif suivant l'une des revendications 4 à 7, caractérisé par le fait qu'il est prévu comme source de lumière (2) une diode à luminescence (LED).

9. Dispositif suivant l'une des revendications 4 à 8, caractérisé par le fait qu'il est prévu comme source de lumière (2) une diode laser à semiconducteurs.

10. Dispositif suivant l'une des revendications 6 à 9, caractérisé par le fait que le modulateur (6) comporte un cristal de Faraday (8) formé d'un composé ($A_{III}$, $B_V$).

11. Dispositif suivant la revendication 10, caractérisé par le fait qu'il est prévu un cristal de Faraday (8) formé de phosphure de gallium GaP.

12. Dispositif suivant la revendication 10, caractérisé par le fait qu'il est prévu un cristal de Faraday (8) formé d'arseniure de gallium GaAs.

13. Dispositif suivant l'une des revendications 6 à 9, caractérisé par le fait que le modulateur (6) comporte un cristal de Faraday (8) qui est formé d'un composé ($A_{1IIIx}A_{2III(1-x)}B_{1Vy}B_{2V(1-y)}$) pour lequel on a $0 \le (x, y) \le 1$.

14. Dispositif suivant l'une des revendications 6 à 13, caractérisé par le fait que la source de lumière (2), le polariseur (4) et le cristal de Faraday (8) sont réunis entre eux par des couches d'immersion.

15. Dispositif suivant l'une des revendications 4 à 14, caractérisé par le fait qu'il est prévu des lentilles pour la formation de l'image de la source de lumière (2) sur le détecteur (16).

16. Dispositif suivant l'une des revendications 6 à 15, caractérisé par le fait qu'il est prévu un cristal de Faraday (8) possédant une surface arrière convexe (9).

17. Dispositif suivant l'une des revendications 6 à 16, caractérisé par le fait qu'il est prévu un grenat en tant que cristal de Faraday.

18. Dispositif suivant la revendication 17, caractérisé par un grenat à base de gadolinium-gallium.

19. Dispositif suivant la revendication 17, caractérisé par un grenat de fer du type $M_3Fe_5O_{12}$, dans lequel M désigne l'une des terres rares, notamment l'ytterbium Yb ou le thulium Tm ou l'yttrium Y ou l'erbium Er ou le holmium Ho.

20. Dispositif suivant la revendication 19, caractérisé par le fait qu'il est prévu comme source de lumière (2) une diode à luminescence émettant dans l'infrarouge, formée de $Ga_xIn_{1-x}As_yP_{1-y}$, possédant une longueur d'onde d'émission $\lambda$ dans la gamme $0,9\ \mu m < \lambda < 1,4\ \mu m$.

21. Dispositif suivant la revendication 20, caractérisé par une longueur d'onde d'émission $\lambda$ dans la gamme $1\ \mu m \le \lambda \le 1,11\ \mu m$.

22. Dispositif suivant la revendication 20 ou 21, caractérisé par le fait que le détecteur (16) est constitué par une photodiode au germanium ou par un élément photoélectrique au germanium.

23. Dispositif suivant l'une des revendications 4 à 22, caractérisé par le fait qu'il est prévu comme tension de commande ($U_{21}$) possédant la fréquence de modulation ($f_F$) pour le modulateur (6), une tension rectangulaire symétrique.

FIG 1

FIG 12

FIG 2

FIG 3

FIG 4

FIG 5

FIG 6

FIG 7

FIG 8

FIG 9

FIG 10

FIG 11

**FIG 13**